## Europäisches Patentamt
## European Patent Office
## Office européen des brevets

(19)

(11) Publication number: **0 104 672**
**B1**

(12)

# EUROPEAN PATENT SPECIFICATION

(45) Date of publication of patent specification: **27.08.86**

(51) Int. Cl.⁴: **C 07 C 1/04, B 01 J 23/74,**
**B 01 J 37/00**

(21) Application number: **83201013.6**

(22) Date of filing: **06.07.83**

(54) **Process for the preparation of hydrocarbons.**

(30) Priority: **02.08.82 NL 8203066**

(43) Date of publication of application:
**04.04.84 Bulletin 84/14**

(45) Publication of the grant of the patent:
**27.08.86 Bulletin 86/35**

(84) Designated Contracting States:
**AT BE DE FR IT NL SE**

(56) References cited:
**DE-B-1 020 613**
**DE-B-2 511 967**
**GB-A-2 077 289**

(73) Proprietor: **SHELL INTERNATIONALE**
**RESEARCH MAATSCHAPPIJ B.V.**
**Carel van Bylandtlaan 30**
**NL-2596 HR Den Haag (NL)**

(72) Inventor: **Hoek, Arend**
**Badhuisweg 3**
**NL-1031 CM Amsterdam (NL)**
Inventor: **Joustra, Annie Hendrika**
**Badhuisweg 3**
**NL-1031 CM Amsterdam (NL)**
Inventor: **Minderhoud, Johannes Kornelis**
**Badhuisweg 3**
**NL-1031 CM Amsterdam (NL)**
Inventor: **Post, Martin Franciscus Maria**
**Badhuisweg 3**
**NL-1031 CM Amsterdam (NL)**

(74) Representative: **Aalbers, Onno et al**
**P.O. Box 302**
**NL-2501 CH The Hague (NL)**

Courier Press, Leamington Spa, England.

## Description

The invention relates to a process for the preparation of hydrocarbons by catalytic reaction of carbon monoxide with hydrogen by contacting a feed comprising $H_2$ and CO at elevated temperature and pressure with a catalyst which has been prepared from a composition comprising a porous carrier material, one or more water-soluble cobalt salts and a quantity of water, by drying, calcination and reduction. Said process is known from British patent application specification 2,077,289 wherein the catalyst is prepared by impregnation.

Surprisingly, it has been found that cobalt catalyst having improved stability can be prepared, when the deposition of the cobalt on the carrier is carried out by kneading instead of impregnation.

Therefore, the present invention is related to a process for the preparation of hydrocarbons by catalytic reaction of carbon monoxide with hydrogen by contacting a feed comprising $H_2$ and CO at elevated temperature and pressure with a catalyst which has been prepared from a composition comprising a porous carrier material, one or more water-soluble cobalt salts and a quantity of water, by drying, calcination and reduction, characterized in that the volume of the quantity of water corresponds with 110—190% of the pore volume of the carrier material and that the composition is a paste which prior to the drying has been kneaded.

The catalyst preparation by kneading, which is similar to the preparation by impregnation carried out by contacting a porous carrier with one or more compounds of the catalytically active metals in the presence of a liquid, followed by the removal of the liquid and calcination of the composition, but before and/or during the removal of the liquid the composition is subjected to an intensive mechanical treatment such as pressing, squeezing or wringing, which generally has as a result that a substantial decrease of the particle size of the carrier material occurs and that the composition takes on the consistency of a paste. Generally several hours' kneading in a suitable kneading machine is sufficient to achieve the desired homogeneous dispersion of the components over the mixture. The intensive mechanical treatment in which a substantial decrease of the particle size of the carrier material occurs, forms the principle difference between the kneading route and the impregnation route. It is true that in the preparation of a catalyst by impregnation a stage may be passed in which the composition contains an amount of liquid corresponding to that present in the above-mentioned paste and that—e.g. by stirring—some mechanical energy may be supplied to the composition, but as a rule the particle size of the carrier material remains substantially unchanged in the catalyst preparation by impregnation. The cobalt catalysts prepared by kneading, which, like the catalysts prepared by impregnation are used in the reduced form, show high activity and $C_3^+$ and $C_5^+$ selectivity, comparable to those of the catalysts prepared by impregnation. The present invention is highly surprising, since comparative research has revealed that in the case of the closely related iron catalysts, replacement of the preparation by impregnation with preparation by kneading leads to a severe drop in stability.

The catalysts used in the process according to the invention comprise cobalt supported on a porous carrier material. As carrier materials both amorphous and crystalline materials are eligible. Suitable carriers include silica and alumina as well as combinations thereof and also zeolites, such as mordenite, faujasite and zeolite-omega. Preference is given to the use of silica as the carrier. The quantities of cobalt present on the catalysts may vary within wide ranges. Preference is given to the use of catalysts containing 10—40 pbw of cobalt per 100 pbw of carrier material. The catalysts used in the process according to the invention preferably include one or more promoters. Suitable promoters for the present cobalt catalysts include magnesium, thorium and zinc. Preference is given to the use of catalysts comprising zirconium, titanium or chromium as promoter. Special preference is given to the use of zirconium as promoter. The quantity of promoter preferably is 0.25—5 pbw of promoter per 100 pbw of carrier material.

In the process according to the invention it is essential that the catalyst used is a catalyst of which the cobalt has been deposited on the carrier by kneading. If the catalyst, in addition to cobalt, comprises a promoter, such as zirconium, this promoter may be deposited on the carrier by kneading, like the cobalt, or by other methods, for instance by impregnation. If the promoter is deposited on the carrier by kneading, preference is given in the catalyst preparation to the use of a starting material which, in addition to the porous carrier, the cobalt compound and the liquid, comprises a compound of the promoter metal. In the catalyst preparation by kneading preference is given to contacting the porous carrier with a solution of the metal compounds concerned in a solvent. A preferred solvent is water. The amount of liquid which may be used in the preparation of catalysts by kneading may vary within wide limits. Amounts of liquid which are smaller, equal to or larger than the pore volume of the carrier come into consideration, provided that during kneading such an amount of liquid is present that under the influence of the intensive mechanical treatment the carrier material, together with the metal compound, can yield a composition with the desired paste-like consistency. A possible excess of liquid may be removed from the composition by evaporation before or during kneading. Preferably the quantity of liquid used in the kneading has a volume which corresponds to 110—190% of the pore volume of the carrier material.

At the end of the kneading the liquid is removed from the paste and the composition is calcined and reduced. The calcination is preferably carried out at a temperature between 350 and 700°C and the reduction at a temperature between 200 and 350°C.

The process according to the invention is applied to a feed comprising $H_2$ and CO. This feed may have been obtained, for instance, from a heavy carbon-containing material such as coal by gasification, or from light hydrocarbons such as natural gas by steam reforming or partial oxiation. The process is preferably applied to a feed whose $H_2$/CO molar ratio lies above 1.75. If the feed available for the process according to the invention has a $H_2$/CO molar ratio lower than 1.75, the latter is preferably increased to have a value higher than 1.75 and in particular between 1.75 and 2.25, before the feed is contacted with the cobalt-catalyst. Increasing the $H_2$/CO molar ratio of low-hydrogen feeds may be effected, inter alia, by addition of hydrogen, removal of carbon monoxide, mixing with a hydrogen-rich $H_2$/CO mixture or by adding water to the low-hydrogen feed and subjecting the mixture to CO-shift.

The process according to the invention is preferably carried out at a temperature of 125—350°C and in particular to 175—275°C and a pressure of 5—150 bar and in particular of 10—100 bar.

A feed very suitable for the process according to the invention is a fraction comprising unconverted carbon monoxide and hydrogen, which fraction can be separated from a reaction product obtained when a $H_2$/CO mixture (1) is contacted with a catalyst comprising one or more metal components having catalytic activity for the conversion of a $H_2$/CO mixture into hydrocarbons and/or oxygen-containing organic compounds.

If the conversion of the $H_2$/CO mixture (1) is carried out with the object of preparing aromatic hydrocarbon, preference is given to the use of a bifunctional catalyst combination comprising one or more metal components having catalytic activity for the conversion of a $H_2$/CO mixture into acyclic hydrocarbons and/or acyclic oxygen-containing organic compounds and a crystalline metal silicate which, after one hour's calcination in air at 500°C, has the following properties:

a) an X-ray powder diffraction pattern in which the strongest lines are the four lines mentioned in Table A,

TABLE A

d(Å)

---

11.1±0.2

10.0±0.2

3.84±0.07

3.72±0.06,     and

b) in the formula which represents the composition of the silicate expressed in moles of the oxides in and in which, in addition to $SiO_2$, one or more oxides of a trivalent metal A, chosen from the group formed by aluminium, iron, gallium, rhodium, chromium and scandium, are present, the $SiO_2/A_2O_3$ molar ratio (m) is higher than 10.

The crystalline metal silicate preferably includes only one metal A chosen from the group formed by aluminium, iron and gallium, and m preferably has a value lower than 1000. Very suitable catalyst combinations for the present purpose are mixtures of the crystalline metal silicate and a catalyst chosen from the group formed by

a) catalysts capable of converting a $H_2$/CO mixture substantially into methanol and/or dimethyl ether,

b) catalysts which contain 30—75 pbw of iron and 5—40 pbw of magnesium per 100 pbw of alumina and which have been prepared by impregnation of an alumina carrier with one or more aqueous solutions of salts of iron and of magnesium, followed by drying of the composition, calcination at a temperature of 700—1200°C and reduction, and

c) catalysts which contain 10—40 pbw of iron and 0.25—10 pbw of chromium per 100 pbw of silica and which have been prepared by impregnation of a silica carrier with one or more aqueous solutions of salts of iron and of chromium, followed by drying of the composition, calcination and reduction at a temperature of 350—750°C.

If the conversion of the $H_2$/CO mixture (1) is carried out with the object of preparing aromatic hydrocarbons, this conversion is preferably carried out at a temperature of 200—500°C and in particular of 250—450°C, a pressure of 1—150 bar and in particular of 5—100 bar and a space velocity of 50—5000 and in particular of 300—3000 Nl gas/l catalyst hour.

If the conversion of the $H_2$/CO mixture (1) is carried out with the object of preparing paraffinic hydrocarbons, the catalyst used is preferably a $Fe/Mg/Al_2O_3$ or $Fe/Cr/SiO_2$ catalyst as described hereinbefore under b) and c).

If the conversion of the $H_2$/CO mixture (1) is carried out with the object of preparing paraffinic hydrocarbons, this conversion is preferably carried out at a temperature of 200—350°C and in particular of 250—350°C, a pressure of 10—70 bar and in particular of 20—50 bar and a space velocity of 500—5000 and in particular of 500—2500 Nl gas/l catalyst/hour.

If the conversion of the $H_2$/CO mixture (1) is carried out with the object of preparing oxygen-containing organic compounds, the catalyst preferably used is a catalyst capable of converting a $H_2$/CO mixture substantially into methanol or dimethyl ether. Examples of suitable catalysts having the property of converting a $H_2$/CO mixture substantially into methanol are catalysts comprising:

1) zinc oxide and chromium oxide,
2) copper, zinc oxide and chromium oxide,

3) copper, zinc oxide and aluminium oxide, and
4) copper, zinc oxide and oxides of rare earths.

Examples of suitable catalysts having the property of converting a $H_2/CO$ mixture substantially into dimethyl ether are catalysts comprising one of the methanol synthesis functions mentioned under 1)—4) and in addition an acid function, such as a physical mixture of gamma-alumina and a composition comprising copper, zinc oxide and chromium oxide.

If the conversion of the $H_2/CO$ mixture (1) is carried out with the object of preparing oxygen-containing organic compounds, this conversion is preferably carried out at a temperature of 175—330°C and in particular of 225—325°C and a pressure of 30—300 bar and in particular of 50—150 bar. The oxygen-containing organic compounds which can be prepared from the $H_2/CO$ mixture (1), may very suitably be used as starting material for catalytic conversion into lower olefins and/or aromatic hydrocarbons. Catalysts very suitable for the purpose are the crystalline metal silicates described hereinbefore.

If the process according to the invention is used as the second step in the two-step process for the preparation of hydrocarbons and/or oxygen-containing organic compounds from a $H_2/CO$ mixture (1), then unconverted hydrogen and carbon monoxide present in the reaction product from the first step are used, together with other components from that reaction product, if desired, as the feed for the second step. Optionally, the total reaction product from the first step may be used as the feed for the second step. In the two-step process an increase of the $H_2/CO$ molar ratio of the feed for the second step can very suitably be effected by mixing the low-hydrogen feed for the second step with a hydrogen-rich $H_2/CO$ mixture which has been prepared by separating a portion from the $H_2/CO$ mixture (1) available as the feed for the first step, mixing this portion with water and contacting the mixture at a temperature above 325°C with a catalyst having CO-shift activity.

In addition to the fact that it can serve as the second step in the afore-mentioned two-step process for the preparation of hydrocarbons and/or oxygen-containing organic compounds from the $H_2/CO$ mixtures, the process according to the invention is also very suitable for use as the first step in a two-step process for the preparation of middle distillates from $H_2/CO$ mixtures. To this end the first step of the two-step process should be carried out by using a cobalt catalyst comprising titanium, zirconium or chromium as a promoter and silica as the carrier and at least the part of the reaction product of the first step whose initial boiling point lies above the final boiling point of the heaviest middle distillate desired as end product should be subjected to a catalytic hydrotreatment in the second step. The catalytic hydrotreatment is carried out by contacting said fraction of the reaction product from the first step at elevated temperature and pressure and in the presence of hydrogen with a catalyst comprising one or more metals having hydrogenation activity supported on a carrier. In the catalytic hydrotreatment preference is given to the use of a catalyst comprising one or more noble metals from Group VIII supported on a carrier. Special preference is given to a catalyst comprising platinum on a carrier, 13—15 %w of which consists of alumina and the rest of silica. The catalytic hydrotreatment is preferably carried out at a temperature of 175—400°C and in particular of 250—350°C, a hydrogen partial pressure of 10—250 bar and in particular of 25—150 bar, a space velocity of 0.1—5 $kg \cdot l^{-1} \cdot h^{-1}$ and in particular of 0.25—2 $kg \cdot l^{-1} \cdot h^{-1}$ and a hydrogen/oil ratio of 100—5000 $Nl \cdot kg^{-1}$ and in particular of 250—2500 $Nl \cdot kg^{-1}$.

If in the afore-mentioned two-step process in which the cobalt catalyst is used in the second step the catalyst used is a cobalt catalyst comprising titanium, zirconium or chromium as a promoter and silica as the carrier, the afore-mentioned catalytic hydrotreatment for the preparation of middle distillates may also very suitably be applied to the heavy reaction product from the second step. The invention is now elucidated with the aid of the following example.

Example
Seven catalysts (catalysts 1—7) were prepared as follows:

Catalyst 1 (Fe/Cu/K/Mg/Al$_2$O$_3$)
This catalyst was prepared starting from an alumina carrier containing 20 pbw of magnesium per 100 pbw of alumina. The preparation was carried out by using dry co-impregnation of the carrier with an aqueous solution comprising iron nitrate, copper nitrate and potassium nitrate. The impregnation was carried out in several stages. After each impregnation stage the composition was dried and calcined in air at 500°C. After the final calcination the composition was reduced in hydrogen at 280°C. Catalyst 1 contained 50 pbw of iron 2.5 pbw of copper, 4 pbw of potassium and 20 pbw of magnesium per 100 pbw of alumina.

Catalyst 2 (Fe/Cu/K/Mg/Al$_2$O$_3$)
This catalyst was prepared starting from the same magnesium-containing alumina carrier as used in the preparation of catalyst 1. The preparation was carried out by kneading a mixture comprising the carrier, water, iron nitrate, copper nitrate and potassium nitrate. The mixture contained a quantity of water corresponding with 150 %v of the pore volume of the carrier. After 3.5 hours' kneading the kneaded mass was dried, calcined in air at 500°C and reduced in hydrogen at 280°C. Catalyst 2 had the same composition as catalyst 1.

Catalyst 3 (Co/SiO$_2$)
This catalyst was prepared by using dry impregnation of a silica carrier with an aqueous solution comprising cobalt nitrate. The impregnation was carried out in several stages. After each impregnation stage the composition was dried and calcined in air at 500°C. After the final calcination the composition was reduced in hydrogen at 250°C. Catalyst 3 contained 25 pbw of cobalt per 100 pbw of silica.

Catalyst 4 (Co/SiO₂)

This catalyst was prepared starting from the same silica carrier as used in the preparation of catalyst 3. The preparation was carried out by kneading a mixture comprising the carrier, water and cobalt nitrate. The mixture contained a quantity of water corresponding with 150 %v of the pore volume of the carrier. After 2.5 hours' kneading the kneaded mass was dried, calcined in air at 500°C and reduced in hydrogen at 250°C. Catalyst 4 had the same composition as catalyst 3.

Catalyst 5 (Co/Zr/SiO₂)

This catalyst was prepared by using dry co-impregnation of a silica carrier with an aqueous solution comprising cobalt nitrate and zirconyl chloride. The impregnation was carried out in several stages. After each impregnation stage the composition was dried and calcined in air at 500°C. After the final calcination the composition was reduced in hydrogen at 250°C. Catalyst 5 contained 25 pbw of cobalt and 0.9 pbw of zirconium per 100 pbw of silica. The catalyst contained 121 mg cobalt per ml catalyst and had a surface area of 17 m² per ml catalyst.

Catalyst 6 (Co/Zr/SiO₂)

This catalyst was prepared starting from the same silica carrier as used in the preparation of catalyst 5. The preparation was carried out by kneading a mixture comprising the carrier, water and cobalt nitrate. The mixture contained a quantity of water corresponding with 150 %v of the pore volume of the carrier. After 3.5 hours' kneading the kneaded mass was dried and calcined in air at 500°C. Subsequently the calcined composition was subjected to dry impregnation with an aqueous solution of zirconyl chloride. After the impregnation thx composition was dried, calcined in air at 500°C and reduced in hydrogen at 250°C. Catalyst 6 contained 25 pbw of cobalt and 0.9 pbw of zirconium per 100 pbw of silica. The catalyst contained 130 mg cobalt per ml catalyst and had a surface area of 15 m² per ml catalyst.

Catalyst 7 (Co/Zr/SiO₂)

This catalyst was prepared in substantially the same way as catalyst 6, with the distinction that in the impregnation stage the aqueous solution used had a higher zirconium concentration. Catalyst 7 contained 25 pbw of cobalt and 1.8 pbw of zirconium per 100 pbw of silica. The catalyst contained 134 mg cobalt per ml catalyst and had a surface area of 15 m² per ml catalyst.

Of the catalysts 1—7 described hereinbefore only catalysts 4, 6 and 7 are eligible for use in the process according to the invention. The other catalysts fall outside the scope of the invention. They have been included for comparison.

Catalysts 1—7 were used in eleven experiments (Experiments 1—11) in the preparation of hydrocarbons from mixtures of carbon monoxide and hydrogen. The experiments were carried out in a 50 ml reactor containing a fixed catalyst bed of 7.5 ml volume.

The conditions under which the experiments were carried out are given in Table B. The results of the experiments are given in Table C.

Of the experiments mentioned in Tables B and C only experiments 4, 7, 8, 10 and 11 are experiments according to the invention. The other experiments fall outside the scope of the invention. They have been included in the patent application for comparison.

Comparison of the results tabulated in Table C with regard to the use of cobalt catalysts and iron catalysts prepared by impregnation and by kneading for preparing hydrocarbons from H₂/CO mixtures, clearly shows the advantage using cobalt catalysts prepared by kneading (both promoted and unpromoted), as opposed to using iron catalysts.

Catalytic hydrotreatment

An Experiment 12 was carried out in which the C₅⁺ fraction of the product obtained according to Experiment 6 was passed together with hydrogen through a 50-ml reactor containing a fixed catalyst bed, at a temperature of 345°C, a pressure of 130 bar, a space velocity of 1.25 l.l⁻¹·h⁻¹ and a hydrogen/oil ratio of 2000 Nl·⁻¹. The catalyst was a Pt/SiO₂-Al₂O₃ catalyst containing 0.82 parts by weight platinum per 100 pbw of carrier, which carrier consisted of 14.6% by weight of alumina and 85.4% by weight of silica. The results of Experiment 12 are given in Table D.

From the results given in Table D it appears that when a catalytic hydrotreatment is applied to a product prepared according to the invention, a considerable part of the 400°C⁺ fraction is converted (a decrease from 29 to 7 %w) and a considerable quantity of 150—360°C fraction is formed (an increase from 48 to 67 %w), whereas only very little 150°C⁻ fraction is formed (an increase from 16 to 20 %w).

TABLE B

| Experiment No. | 1 | 2 | 3 | 4 | 5 | 6 | 7 | 8 | 9 | 10 | 11 |
|---|---|---|---|---|---|---|---|---|---|---|---|
| Catalyst No. | 1 | 2 | 3 | 4 | 5 | 5 | 6 | 6 | 5 | 6 | 7 |
| Temperature, °C | 280 | | 220 | | 220 | 250 | 220 | 235 | 215 | | 220 |
| Pressure, bar | 30 | | 20 | | 30 | | | | 20 | | 30 |
| Space velocity, l.l$^{-1}$·h$^{-1}$ | 1000 | | 500 | | 600 | | | | 1000 | | 600 |
| H$_2$/CO molar ratio of feed | 0.5 | | 2.0 | | 2.0 | | | | 3.0 | | 2.0 |

TABLE C

| Experiment No. | | 1 | | 2 | | 3 | | 4 | | 5 | | 6 | | 7 | | 8 | | 9 | | 10 | | 11 | |
|---|---|---|---|---|---|---|---|---|---|---|---|---|---|---|---|---|---|---|---|---|---|---|---|
| Conversion of $H_2/CO$ mixture | Moment of measuring (run hour) | 145 | 670 | 193 | 462 | 10 | 100 | 10 | 180 | 10 | 200 | 200 | 300 | 10 | 300 | 300 | 1000 | 10 | 150 | 10 | 150 | 10 | 300 |
| | Conversion, %v | 90 | 80 | 81 | 60 | 33 | 15 | 30 | 19 | 85 | 40 | 96 | 71 | 80 | 65 | 94 | 73 | 86 | 65 | 70 | 65 | 75 | 61 |
| Decrease in conversion per 100 hours, %v | | 2 | | 8 | | 20 | | 6 | | 24 | | 25 | | 5 | | 3 | | 15 | | 4 | | 5 | |
| Selectivity calculated on $C_1^+$, %w | $C_3^+$ selectivity | 94 | | 93 | | 87 | | 87 | | 89 | | 73 | | 88 | | 79 | | 84 | | 84 | | 90 | |
| | $C_5^+$ selectivity | 88 | | 84 | | 76 | | 75 | | 79 | | 65 | | 80 | | 68 | | 74 | | 73 | | 82 | |

# 0 104 672

TABLE D

| Composition, %w | $C_1^+$ product of Experiment 6 | $C_5^+$ fraction of the $C_1^+$ product of Experiment 6 | $C_1^+$ product after the catalytic hydrotreatment |
|---|---|---|---|
| $C_4^-$ | 25 | — | 2 |
| $C_5$—150°C | 12 | 16 | 18 |
| 150—250°C | 19 | 25 | 33 |
| 250—360°C | 17 | 23 | 34 |
| 360—400°C | 5 | 7 | 6 |
| 400°C$^+$ | 22 | 29 | 7 |

## Claims

1. A process for the preparation of hydrocarbons by catalytic reaction of carbon monoxide with hydrogen by contacting a feed comprising $H_2$ and CO at elevated temperature and pressure with a catalyst which has been prepared from a composition comprising a porous carrier material, one or more water-soluble cobalt salts and a quantity of water, by drying, calcination and reduction, characterized in that the volume of the quantity of water corresponds with 110—190% of the pore volume of the carrier material and that the composition is a paste which prior to the drying has been kneaded.

2. A process as claimed in claim 1, characterized in that the catalyst comprises silica as carrier material.

3. A process as claimed in claim 1 and/or 2, characterized in that the catalyst contains 10—40 pbw of cobalt per 100 pbw of carrier material.

4. A process as claimed in any one of claims 1—3, characterized in that the catalyst comprises a promoter chosen from the group formed by zirconium, titanium and chromium in a quantity of 0.25—5 pbw per 100 pbw of carrier material.

5. A process as claimed in any one of claims 1—4, characterized in that the catalyst is calcined at a temperature between 350 and 700°C and reduced at a temperature between 200—350°C.

6. A process as claimed in any one of claims 1—5, characterized in that it is applied to a feed comprising $H_2$ and CO whose $H_2$/CO molar ratio lies between 1.75 and 2.25.

7. A process as claimed in any one of claims 1—6, characterized in that it is carried out at a temperature of 125—350°C and a pressure of 5—150 bar.

8. A process as claimed in any one of claims 1—7, characterized in that it is used as the first step of a two-step process for the preparation of middle distillates from a $H_2$/CO mixture, in which the catalyst used in the first step is a cobalt catalyst comprising titanium, zirconium or chromium as a promoter and silica as the carrier and in which at least the part of the reaction product of the first step whose initial boiling point lies above the final boiling point of the heaviest middle distillate desired as end product is subjected to a catalytic hydrotreatment in the second step.

## Patentansprüche

1. Verfahren zur Herstellung von Kohlenwasserstoffen durch katalytische Reaktion von Kohlenmonoxid mit Wasserstoff durch Inkontaktbringen eines Wasserstoff und Kohlenmonoxid enthaltenden Einsatzmaterials bei erhöhter Temperatur und erhöhtem Druck mit einem Katalysator, der aus einer ein poröses Trägermaterial, ein oder mehrere wasserlösliche Kobaltsalze und eine Menge an Wasser enthaltenden Zusammensetzung durch Trocknen, Calcinieren und Reduzieren erhalten worden ist, dadurch gekennzeichnet, daß das Volumen der Menge an Wasser 110 bis 190% des Porenvolumens des Trägermaterials entspricht und daß die Zusammensetzung eine Paste ist, welche vor dem Trocknen geknetet worden ist.

2. Verfahren nach Anspruch 1, dadurch gekennzeichnet, daß der Katalysator Siliziumdioxid als Trägermaterials umfaßt.

3. Verfahren nach Anspruch 1 und/oder 2, dadurch gekennzeichnet, daß der Katalysator 10 bis 40 Gew.-Teile Kobalt je 100 Gew.-Teile Trägermaterial enthält.

4. Verfahren nach einem der Ansprüche 1 bis 3, dadurch gekennzeichnet, daß der Katalysator einen Promotor, ausgewählt aus der aus Zirkon, Titan und Chrom gebildeten Gruppe, in einer Menge von 0,25 bis 5 Gew.-Teile je 100 Gew.-Teile Trägermaterial umfaßt.

5. Verfahren nach einem der Ansprüche 1 bis 4, dadurch gekennzeichnet, daß der Katalysator bei einer Temperatur zwischen 350 und 700°C calciniert und bei einer Temperatur zwischen 200 und 350°C reduziert wird.

6. Verfahren nach einem der Ansprüche 1 bis 5, dadurch gekennzeichnet, daß es auf ein Wasserstoff und Kohlenmonoxid umfassendes Einsatz-

8

material angewendet wird, dessen $H_2$/CO-Molver-
hältnis zwischen 1,75 und 2,5 beträgt.

7. Verfahren nach einem der Ansprüche 1 bis 6, dadurch gekennzeichnet, daß es bei einer Temperatur von 125 bis 350°C und bei einem Druck von 5 bis 150 bar ausgeführt wird.

8. Verfahren nach einem der Ansprüche 1 bis 7, dadurch gekennzeichnet, daß es als erste Stufe eines Zweistufenverfahrens zur Herstellung von Mitteldestillaten aus einem $H_2$/CO-Gemisch ange-wendet wird, worin der in der ersten Stufe verwendete Katalysator ein Titan, Zirkon oder Chrom als Promotor und Siliziumdioxid als Träger umfassender Kobaltkatalysator ist und worin wenigstens jener Teil des Reaktionsproduktes der ersten Stufe, dessen Anfangssiedepunkt über dem Endsiedepunkt des schwersten, als Endprodukt gewünschten Mitteldestillates liegt, in der zweiten Stufe einer katalytischen Wasserstoffbehandlung unterworfen wird.

**Revendications**

1. Un procédé pour la préparation d'hydrocarbures par réaction catalytique d'oxyde de carbone avec l'hydrogène par mise en contact d'une charge contenant $H_2$ et CO à température et pression élevées avec un catalyseur qui a été préparé à partir d'une composition comprenant une matière poreuse de support, un ou plusieurs sels de cobalt solubles dans l'eau et une certaine quantité d'eau, par séchage, calcination et réduction, caractérisé en ce que le volume de la quantité d'eau correspond à 110—190% du volume des pores de la matière de support et que la composition est une pâte qui avant le séchage a été malaxée.

2. Un procédé selon la revendication 1, caractérisé en ce que le catalyseur comprend de la silice comme matière de support.

3. Un procédé selon les revendications 1 et/ou 2, caractérisé en ce que le catalyseur contient 10—40 parties en poids de cobalt pour 100 parties en poids de matière de support.

4. Un procédé selon l'une quelconque des revendications 1 à 4, caractérisé en ce que le catalyseur comprend un promoteur choisi dans le groupe formé par le zirconium, le titane et pe chrome en une quantité de 0,25—5 parties en poids pour 100 parties en poids de matière de support.

5. Un procédé selon l'une quelconque des revendications 1 à 4, caractérisé en ce que le catalyseur est calciné à une température comprise entre 350 et 700°C et réduit à une température comprise entre 200 et 350°C.

6. Un procédé selon l'une quelconque des revendications 1 à 5, caractérisé en ce qu'il est appliqué à une charge comprenant $H_2$ et CO dont le rapport molaire $H_2$/CO est compris entre 1,75 et 2,25.

7. Un procédé selon l'une quelconque des revendications 1 à 6, caractérisé en ce qu'il est mis en oeuvre à une température de 125—350°C et une pression de 5—150 bars.

8. Un procédé selon l'une quelconque des revendications 1 à 7, caractérisé en ce qu'il est utilisé comme première étape d'un procédé à deux étapes pour la préparation de distillats moyens à partir d'un mélange $H_2$/CO, dans lequel le catalyseur utilisé dans la première étape est un catalyseur au cobalt comprenant du titane, du zirconium ou du chrome comme promoteur et de la silice comme support et dans lequel au moins la partie du produit de réaction de la première étape dont le point initial d'ébullition se trouve au-dessus du point final d'ébullition du distillat moyen le plus lourd désiré comme produit final est soumise à un hydrotraitement catalytique dans la seconde étape.